# EUROPEAN PATENT APPLICATION

(11) **EP 4 792 752 A1**
(43) Date of publication of application: **19.08.2026**
(21) Application number: 24876965.5
(22) Date of filing: 30.08.2024
(51) Int. Cl.: A61B 10/00, A61B 5/00, A61B 5/02, A61B 5/0245, A61B 5/288, A61B 5/344, A61B 5/397

(54) **PREGNANCY STATE ESTIMATION DEVICE AND PREGNANCY STATE ESTIMATION METHOD**

(30) Priority: 13.10.2023 JP 2023177749; 22.11.2023 JP 2023198638
(71) Applicant: Nonat Inc., Tokyo, 150-0043 (JP)
(72) Inventor: ITO, Keisuke, Shibuya-Ku, Tokyo 150-0043 (JP); TAKASAKI, Yohsuke, Shibuya-Ku, Tokyo 150-0043 (JP); TAKANO, Kyohei, Shibuya-Ku, Tokyo 150-0043 (JP); TANAKA, Shunsuke, Shibuya-Ku, Tokyo 150-0043 (JP)
(74) Representative: Hoffmann Eitle
(86) International application number: PCT/JP2024/031414
(87) International publication number: WO 2025/079365

(57) **Abstract**

To make it possible to inexpensively and easily ascertain the status of a mother body or a fetus even outside a hospital. A pregnancy status estimation device includes an information acquisition unit that acquires a mother body-derived electrical activity record measured percutaneously, an inference unit that infers a pregnancy status of a mother body by inputting the electrical activity record into a learned model that receives the electrical activity record as input and outputs the pregnancy status including information on at least one of uterine contractions of the mother body and a fetal heartbeat in the mother body, and an output unit that outputs the pregnancy status generated by the inference unit.

## Description

### Field

The present invention relates to a pregnancy status estimation device and a pregnancy status estimation method.

### Background

Technology is known to take measurements from a pregnant mother body and ascertain information on the mother body and a fetus on the basis of data obtained. For example, Patent Literature 1 discloses a childbirth monitoring apparatus that calculates a fetal heart rate on the basis of signals from electrocardiographic electrodes attached to a fetal head after rupture and an ultrasound transducer attached to the surface of the maternal abdomen, and calculates the maternal intrauterine pressure and labor pain intensity on the basis of signals from an internal pressure transducer and a labor pain transducer inserted into the uterus after rupture.

### Citation List

### Patent Literature

Patent Literature 1: Japanese Patent Application Laid-open No. 2006-223335

### Summary

### Technical Problem

In addition to the childbirth monitoring apparatus in Patent Literature 1, there are various other devices capable of ascertaining information on a mother body and a fetus, such as fetal heart rate labor pain chart monitors and Doppler heart rate monitors. However, since all of these devices require specialized knowledge and skills to use and are expensive, the devices are assumed to be installed in medical facilities such as hospitals and used by doctors and other medical personnel.

During pregnancy, attention needs be paid to changes in the status of a mother body and a fetus because of the risk of premature birth and intrauterine fetal death. However, outside of a hospital, at the home of a pregnant woman, the pregnant woman's own subjective symptoms, the sensation of fetal movement, and the like are the only clues, so the means of ascertaining the status of a mother body or a fetus are limited.

In order to enable early identification of signs of premature birth and intrauterine fetal death and early medical intervention, the status of a mother body or a fetus needs be ascertained inexpensively and easily even outside a hospital.

In view of the above, the present disclosure is made for the purpose of providing a pregnancy status estimation device and a pregnancy status estimation method that can inexpensively and easily ascertain the status of a mother body or a fetus even outside a hospital.

### Solution to Problem

A pregnancy status estimation device according to the present disclosure comprising: an information acquisition unit that acquires a mother body-derived electrical activity record measured percutaneously; an inference unit that infers a pregnancy status of a mother body by inputting the electrical activity record into a learned model that receives the electrical activity record as input and outputs the pregnancy status including information on at least one of uterine contractions of the mother body and a fetal heartbeat in the mother body; and an output unit that outputs the pregnancy status generated by the inference unit.

A pregnancy status estimation method according to the present disclosure comprising: a step of acquiring a mother body-derived electrical activity record measured percutaneously, a step of inferring a pregnancy status of the mother body by inputting the electrical activity record to a learned model that receives the electrical activity record as input and outputs the pregnancy status including information on at least one of uterine contractions of a mother body and a fetal heartbeat in the mother body, and
a step of outputting the pregnancy status.

A pregnancy status estimation device according to the present disclosure comprising: an information acquisition unit that acquires a mother body-derived electrical activity record measured percutaneously; an inference unit that infers a pregnancy status of a mother body by inputting the electrical activity record into a learned model that receives the electrical activity record as input and outputs the pregnancy status including information on an in utero movement of a fetus in the mother body; and an output unit that outputs the pregnancy status generated by the inference unit.

A pregnancy status estimation method according to the present disclosure comprising: a step of acquiring a mother body-derived electrical activity record measured percutaneously; a step of inferring a pregnancy status of a mother body by inputting the electrical activity record into a learned model that receives the electrical activity record as input and outputs the pregnancy status including information on an in utero movement of a fetus in the mother body; and a step of outputting the pregnancy status.

### Advantageous Effects of Invention

The present disclosure can inexpensively and easily ascertain the status of a mother body or a fetus even outside a hospital.

### Brief Description of Drawings

FIG. 1 is a schematic block diagram illustrating a pregnancy status estimation device according to a first embodiment.
FIG. 2 is an explanatory diagram illustrating a first learned model.
FIG. 3 is an explanatory diagram illustrating a second learned model.
FIG. 4 is an explanatory diagram for explaining the output of a pregnancy status.
FIG. 5 is an explanatory diagram for explaining the generation (machine learning) of the first learned model.
FIG. 6 is an explanatory diagram for explaining the generation (machine learning) of the second learned model.
FIG. 7 is a flowchart for explaining a pregnancy status estimation method according to an embodiment.
FIG. 8 is an explanatory diagram illustrating a first configuration example of the pregnancy status estimation device according to an embodiment.
FIG. 9 is an explanatory diagram illustrating a second configuration example of the pregnancy status estimation device according to an embodiment.
FIG. 10 is a schematic block diagram illustrating a pregnancy status estimation device according to a second embodiment.
FIG. 11 is an explanatory diagram illustrating a learned model according to the second embodiment.
FIG. 12 is an explanatory diagram for explaining evaluation information according to the second embodiment.
FIG. 13 is an explanatory diagram for explaining the generation (machine learning) of a learned model.
FIG. 14 is a schematic diagram for explaining the generation of an individually learned model by fine tuning according to a modification.
FIG. 15 is a schematic diagram for explaining an inference process using the individually learned model according to the modification.

### Description of Embodiments

### First Embodiment

The following is a description of embodiments of a pregnancy status estimation device and a pregnancy status estimation method according to the present invention on the basis of the drawings. The present invention is not limited by these embodiments. In addition, components in the following embodiments include those that are substitutable and easy for those skilled in the art, or those that are substantially the same. The present invention is not limited by these embodiments, and when there are a plurality of embodiments, the present invention also includes those configured by combining the respective embodiments.

### Pregnancy Status Estimation Device

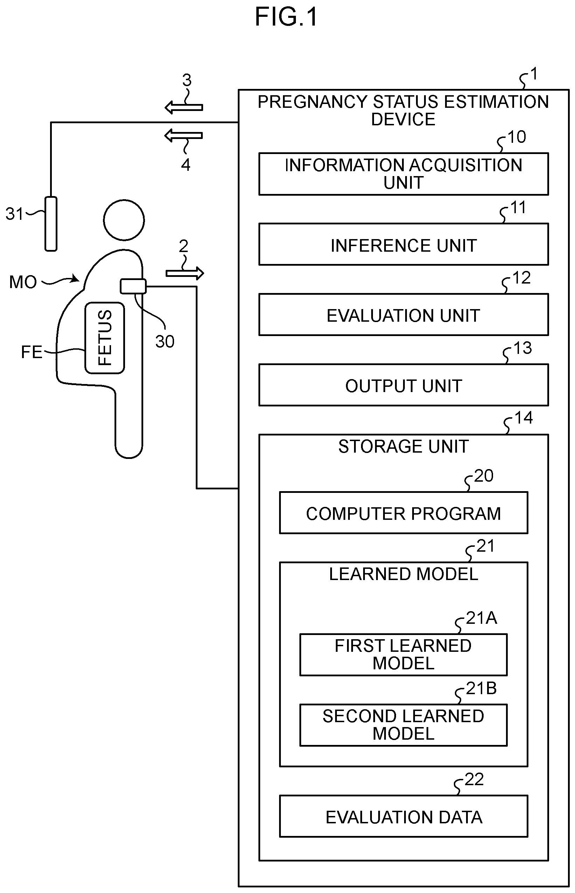

FIG. 1 is a schematic block diagram illustrating a pregnancy status estimation device 1 according to a first embodiment. As illustrated in FIG. 1, the pregnancy status estimation device 1 is a device that infers a pregnancy status 3 of a mother body MO of a pregnant woman on the basis of a mother body MO-derived electrical activity record 2 that is non-invasively and percutaneously acquired from the mother body MO, and outputs the obtained pregnancy status 3. The pregnancy status estimation device 1 is a device that can be used outside of a medical facility such as a hospital, for example, at the home of a pregnant woman.

The pregnancy status 3 is information indicating the status of the mother body MO and/or a fetus FE in the mother body MO. In the present specification, the "pregnancy status" is a broad concept that encompasses the status of the mother body MO and the status of the fetus FE in the mother body MO. In the first embodiment, the status of the mother body MO included in the pregnancy status 3 includes information on uterine contractions of the mother body MO. In the first embodiment, the status of the fetus FE included in the pregnancy status 3 includes information on a fetal heartbeat in the mother body MO. The pregnancy status 3 may include information on signs of perinatal complications such as premature birth, intrauterine fetal death, and placental abruption. The information on the uterine contractions is information that may include the signs of premature birth and placental abruption. The information on the fetal heartbeat is information that may include the signs of intrauterine fetal death. Detecting the signs of perinatal complications from the pregnancy status 3 can be expected to improve early medical intervention for pregnant women.

The mother body MO-derived electrical activity is acquired by a sensor 30 attached to the mother body MO. The sensor 30 non-invasively measures the mother body MO-derived electrical activity. The sensor 30 is a sensor that detects electrical signals used for electrocardiogram or electromyogram, for example. Such a sensor 30 percutaneously measures electrical signals (changes in an electric field) generated by a biological activity of the mother body MO by means of electrodes that contact the mother body MO. The pregnancy status estimation device 1 has an interface that communicates with the sensor 30 by wire or wirelessly, and receives measurement data from the sensor 30.

The pregnancy status estimation device 1 is configured by a computer including a processor such as a central processing unit (CPU) and a memory such as a read only memory (ROM) and a random access memory (RAM). The pregnancy status estimation device 1 includes an information acquisition unit 10, an inference unit 11, an evaluation unit 12, an output unit 13, and a storage unit 14. The information acquisition unit 10, the inference unit 11, the evaluation unit 12, and the output unit 13 are configured as functional blocks implemented on a computer program 20 by a processor executing the computer program 20 stored in the storage unit 14. The computer program 20 is a computer program for causing the computer to function as the information acquisition unit 10, the inference unit 11, the evaluation unit 12, and the output unit 13. The information acquisition unit 10, the inference unit 11, the evaluation unit 12, and the output unit 13 may each be configured by dedicated hardware.

The information acquisition unit 10 acquires the mother body MO-derived electrical activity record 2 measured percutaneously. The information acquisition unit 10 acquires the electrical activity record 2 from the sensor 30 attached to the mother body MO. The electrical activity record 2 is time-series data of the mother body MO-derived electrical activity measured by the sensor 30. The information acquisition unit 10 acquires the electrical activity record 2 from the sensor 30 at predetermined sampling cycles and stores the acquired electrical activity record 2 in the storage unit 14.

The inference unit 11 generates the pregnancy status 3 of the mother body MO on the basis of the mother body MO-derived electrical activity record 2 acquired by the information acquisition unit 10. Specifically, the inference unit 11 infers the pregnancy status 3 of the mother body MO by inputting the electrical activity record 2 into a learned model 21 that receives the electrical activity record 2 as input and outputs the pregnancy status including information on at least one of the uterine contractions of the mother body MO and the fetal heartbeat in the mother body **MO.** The learned model 21 is generated by machine learning using teacher data including the mother body-derived electrical activity record 2 as input and the information on the uterine contractions and/or the fetal heartbeat as output, and is stored in advance in the storage unit 14. The output pregnancy status 3 is numerical information on at least one of the uterine contractions and the fetal heartbeat. The inference unit 11 stores the pregnancy status 3 of the mother body MO, which is the result of inference using the learned model 21, in the storage unit 14.

The evaluation unit 12 generates evaluation information 4 according to the content of the pregnancy status 3 generated by the inference unit 11. The evaluation unit 12 receives the pregnancy status 3 generated by the inference unit 11 as input and outputs the evaluation information 4 corresponding to the input pregnancy status 3. The evaluation information 4 is information indicating the evaluation of the status of the pregnant woman (mother body MO) and the fetus FE ascertained from the pregnancy status 3.

The evaluation unit 12 may generate the evaluation information 4 by using a learned model for evaluation generated by machine learning or by rule-based information processing. As an example of the rule-based information processing, the evaluation unit 12 generates the evaluation information 4 according to the input pregnancy status 3 by using evaluation data 22 that maps the content (numerical values) of the pregnancy status 3 and the evaluation information 4. The evaluation data 22 includes, for example, a table that maps a plurality of numerical ranges (threshold values) that classify the pregnancy status 3 with the evaluation information 4 associated with the respective numerical ranges. The evaluation data 22, for example, has a variable for receiving the value of the pregnancy status 3 and includes a formula for determining the evaluation category of the pregnancy status 3. The evaluation data 22 is prepared in advance on the basis of the information obtained by the pregnancy status 3 and the knowledge of specialists and others in the field.

The output unit 13 outputs the pregnancy status 3 generated by the inference unit 11. The output unit 13 also outputs the evaluation information 4 according to the pregnancy status 3, in addition to the pregnancy status 3. The pregnancy status estimation device 1 can be connected to a display unit 31 by wire or wirelessly via an interface (not illustrated). The display unit 31 may be a display device or a display device provided in an information processing terminal such as a smart phone. The output unit 13 presents these information to pregnant women and others by outputting the pregnancy status 3 and the evaluation information 4 to the display unit 31. Users such as pregnant women and their family members can ascertain the status of the mother body MO and the fetus FE through the pregnancy status 3 while staying at home or other places outside a hospital. The evaluation information 4 allows the user to ascertain the status of the mother body MO and the fetus FE without requiring medical expertise, and to take actions such as consulting a medical institution when necessary. This allows pregnant women to expect to take actions such as early medical examinations based on objective information, even when they do not have subjective symptoms (subjective information).

### Learned Model

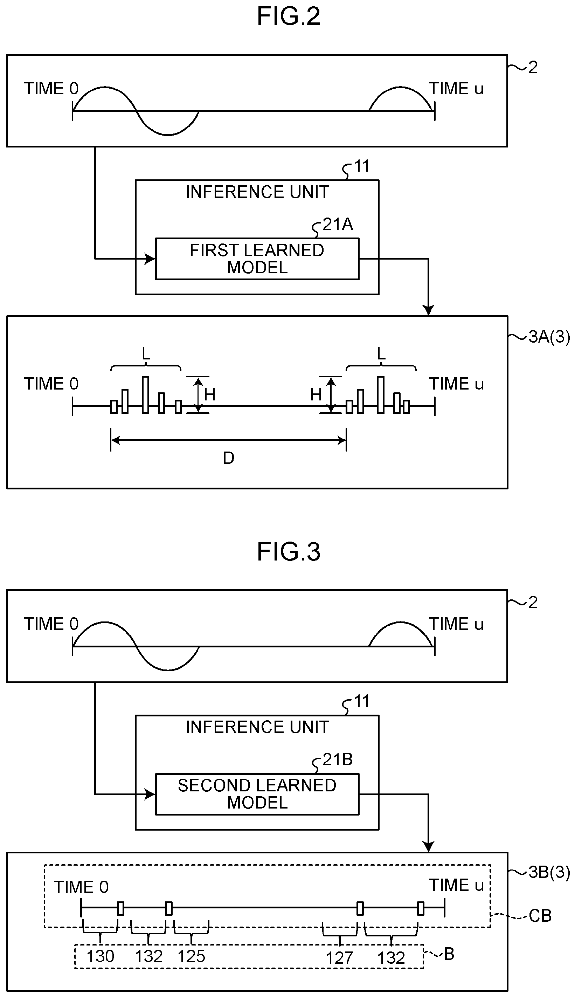

In the first embodiment, the learned model 21 includes a first learned model 21A that outputs information 3A (see FIG. 2) on the uterine contractions of the mother body MO and a second learned model 21B that outputs information 3B (see FIG. 3) on the fetal heartbeat in the mother body MO. The inference unit 11 infers the pregnancy status 3 including the information 3A on the uterine contractions of the mother body MO and the information 3B on the fetal heartbeat in the mother body MO.

FIG. 2 is an explanatory diagram illustrating the first learned model 21A. The first learned model 21A is pre-generated by machine learning so that the mother body MO-derived electrical activity record 2 is used as input and the information 3A on the uterine contractions of the mother body MO is used as output. As illustrated in FIG. 2, the inference unit 11 infers the information 3A on the uterine contractions of the mother body MO by inputting, into the first learned model 21A, the mother body MO-derived electrical activity record 2 acquired by the information acquisition unit 10. The electrical activity record 2 is time-series data of electrical signals measured by the sensor 30. In FIG. 2, the electrical activity record 2 is indicated by a waveform graph in which a vertical axis indicates signal intensity and a horizontal axis indicates time (time u). The first learned model 21A outputs the information 3A on the uterine contractions of the mother body MO as the time-series data of the intensity of uterine contractions (electrical signals derived from uterine contractions). In FIG. 2, the information 3A on the uterine contractions of the mother body MO is indicated by a waveform graph in which a vertical axis indicates the intensity of the uterine contractions and a horizontal axis indicates time (time u).

The information 3A on the uterine contractions includes at least one of the following information: intensity, duration, interval, cycle, frequency, and intensity waveform of the uterine contractions. In the example illustrated in FIG. 2, the information 3A on the uterine contractions includes information on the intensity H, duration L, and interval D of the uterine contractions. The intensity H of the uterine contractions indicates the intensity of the uterine contractions in one uterine contraction. The duration L of the uterine contractions is the time length from the beginning to the end of one uterine contraction. The interval D of the uterine contractions is the time interval between one uterine contraction and the next uterine contraction. Although FIG. 2 illustrates the intensity waveforms (time-series data) of the uterine contractions, the information 3A on the uterine contractions may include only data on the intensity H, duration L, and interval D of the uterine contractions. In the first embodiment, the first learned model 21A outputs the intensity waveform of the uterine contractions, which includes the intensity H, duration L, and interval D of the uterine contractions, as the information 3A on the uterine contractions. The information 3A on the uterine contractions may include either or both the cycle or frequency of the uterine contractions. The cycle of the uterine contractions is the sum of the duration L and the interval D. The frequency of the uterine contractions is the number of occurrences of the uterine contractions per unit time.

FIG. 3 is an explanatory diagram illustrating the second learned model 21B. The second learned model 21B is pre-generated by machine learning so that the mother body MO-derived electrical activity record 2 is used as input and the information 3B on the fetal heartbeat in the mother body MO is used as output. As illustrated in FIG. 3, the inference unit 11 infers the information 3B on the fetal heartbeat in the mother body MO by inputting, into the second learned model 21B, the mother body MO-derived electrical activity record 2 acquired by the information acquisition unit 10. The electrical activity record 2 is the same information as that input to the first learned model 21A. The second learned model 21B outputs the information 3B on the fetal heartbeat as the time-series data of the intensity of the fetal heartbeat (electrical signal derived from the fetal heartbeat). In FIG. 3, the information 3B on the fetal heartbeat is indicated by a waveform graph in which a vertical axis indicates the intensity of the fetal heartbeat and a horizontal axis indicates time (time u).

The information 3B on the fetal heartbeat includes at least one of the following information: fetal heartbeat, fetal heart rate, fetal heart sound, heartbeat interval, heart sound waveform, and findings of the fetal heartbeat. In the example illustrated in FIG. 3, the information 3B on the fetal heartbeat in the mother body MO includes at least one of the following information: fetal heartbeat CB, fetal heart rate B, and fetal heart sound. The fetal heartbeat CB is the time-series data of the intensity of the fetal heartbeat (data of intensity change waveform). The fetal heart rate B is information on the number of beats per unit time, and expressed, for example, in beats per minute (bpm). The "130", "132", "125", "127", and "132" in FIG. 3 indicate numerical examples of the fetal heart rate B calculated at respective time points. The fetal heart sound is data that expresses the intensity change of the fetal heartbeat CB as sound. In the first embodiment, the second learned model 21B outputs the intensity waveform of the fetal heartbeat, including the fetal heartbeat CB and the fetal heart rate B, as the information 3B on the fetal heartbeat. The information 3B on the fetal heartbeat may include one or more of the following: heartbeat interval, heart sound waveform, and findings of the fetal heartbeat. The heartbeat interval is information indicating the regular (equal pulse)/irregular (irregular pulse) rhythm of the fetal heartbeat. The heartbeat waveform is the intensity waveform of the fetal heart sound. The findings of the fetal heartbeat are characteristics that can be read from the heartbeat waveform (electrocardiogram waveform) or the heart sound waveform (ultrasound waveform), and are, for example, information such as blood flow rate, blood flow rate waveform, presence or absence of tachycardia or bradycardia, and degree of variation in heart rate (heartbeat interval).

In the first embodiment, the inference unit 11 may preprocess the electrical activity record 2 that is input to the learned model 21 (the first learned model 21A and the second learned model 21B). The learned model 21 in this case is learned using teacher data from the electrical activity record 2 that has been preprocessed in the same way as input data.

For example, the inference unit 11 may perform preprocessing on the electrical activity record 2, including a filtering process of removing predetermined signal components corresponding to respiratory fluctuations of the mother body MO. This improves the accuracy of the estimation of the pregnancy status 3 because variations caused by the respiration of the mother body MO can be removed from the electrical activity record 2. The filtering process of removing predetermined signal components corresponding to respiratory fluctuations of the mother body MO is a process of removing a frequency component by a frequency-selective filter (for example, a band rejection filter). Respiratory fluctuations included in the electrical activity record 2 generally fall in a band of 0.08 Hz to 1 Hz. The frequency band to be removed is, for example, a range of 0.1 Hz to 0.5 Hz. By setting the frequency band to be removed in this range, the central signal component of the respiratory fluctuations can be effectively removed. In the case of using a band rejection filter, the filter order is preferably set in a range of 2 or higher, and particularly, the filter order is preferably set to a predetermined value in a range of 2 to 4. This allows effective removal of the signal components corresponding to the respiratory fluctuations in the mother body MO without excessively increasing a computational load.

For example, the inference unit 11 may perform preprocessing to extract information identifying at least one of the baseline, P-point, Q-point, R-point, S-point, and T-point from the electrical activity record 2 being electrocardiogram data. Identifying the baseline means specifying a signal intensity indicating the baseline. Identifying the P, Q, R, S, and T-points means specifying pairs of time information indicating such points and signal intensity. The inference unit 11 inputs information identified from the electrocardiogram data into the learned model 21 as a preprocessed electrical activity record 2. For example, when the baseline and the P, Q, R, S, and T-points are all identified, the electrical activity record 2 is time-series data including the signal intensity of the baseline and pairs of the time information indicating the P, Q, R, S, and T-points and the signal intensity. In this way, variations between the P, Q, R, S, and T-points in the electrocardiogram data, and the like, are removed from the electrical activity record 2, and only components that characterize the electrocardiogram waveform can be extracted. In other words, after this preprocessing, the electrical activity record 2 results in feature-extracted data that looks like a line graph with an electrocardiogram waveform composed of the baseline and each point. Therefore, noise components from the electrical activity record 2 can be removed, and a computational load can be effectively reduced in learning and inference by reducing the amount of data. The inference unit 11 may not identify all feature points (P, Q, R, S, and T-points), and may identify only some of them.

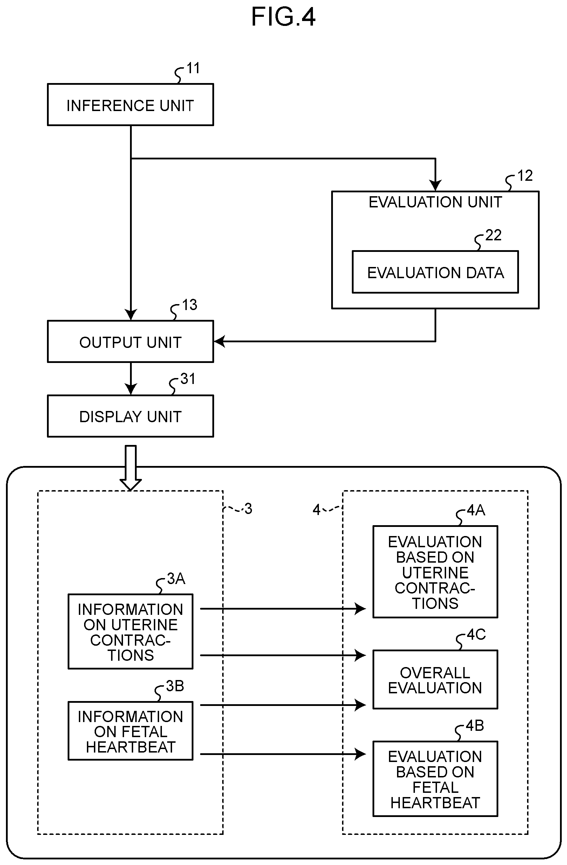

FIG. 4 is an explanatory diagram for explaining the output of the pregnancy status 3. As illustrated in FIG. 4, the data on the pregnancy status 3 generated by the inference unit 11 is supplied to the evaluation unit 12 and the output unit 13.

The evaluation unit 12 generates the evaluation information 4 on the basis of the data on the pregnancy status 3 and the evaluation data 22 stored in advance in the storage unit 14. The evaluation unit 12 generates evaluation information 4A based on the uterine contractions on the basis of the information 3A on the uterine contractions of the mother body MO. The evaluation unit 12 generates evaluation information 4B based on the fetal heartbeat on the basis of the information 3B on the fetal heartbeat in the mother body MO. The evaluation unit 12 further generates overall evaluation information 4C based on both the information 3A on the uterine contractions and the information 3B on the fetal heartbeat. The generated evaluation information 4 includes the evaluation information 4A, the evaluation information 4B, and the overall evaluation information 4C. The evaluation information 4 generated by the evaluation unit 12 is supplied to the output unit 13.

The output unit 13 outputs, to the display unit 31, output data including the pregnancy status 3 generated by the inference unit 11 and the evaluation information 4 generated by the evaluation unit 12. The output unit 13 presents these information to the user by displaying the pregnancy status 3 and the evaluation information 4 on the display unit 31.

In the example of FIG. 4, the output pregnancy status 3 includes the information 3A on the uterine contractions of the mother body MO and the information 3B on the fetal heartbeat in the mother body MO. The output evaluation information 4 is output, for example, in the form of a text (message) explaining the status and advice ascertained from the pregnancy status 3. In addition to text, the evaluation information 4 may also include an indication of the degree of goodness of the pregnancy status 3, for example, an indication in a score format (such as scores from 0 to 100) or in a class format (rank A, rank B, rank C, and the like in descending order of goodness).

The evaluation information 4A based on the uterine contractions includes, for example, an evaluation of the signs or possibility of premature birth or placental abruption. The possibility of premature birth can be evaluated by one of the indicators of the presence or absence of uterine contractions that promote the maturation of the cervical canal (True Labor). True Labor is characterized by the intensity H, duration L, and interval D. In True Labor, an increase in the intensity H and duration L of the uterine contractions is observed over time. In uterine contractions that do not result in the maturation of the cervical canal (False Labor), uterine contractions diminish or cease over time. Determination between True Labor and False Labor requires continuous data acquisition over a certain period of time, but currently no device that can make this determination exist outside a hospital. Even within a hospital, time constraints make it difficult to sufficiently acquire data. In the first embodiment, since the information 3A on the uterine contractions can be acquired and accumulated even outside a hospital, an evaluation index regarding signs of premature birth can be obtained from the changing trends of the intensity H and duration L of the uterine contractions over time, which characterize True Labor. Placental abruption is a highly emergent disease, and early medical intervention improves the perinatal prognosis for mother and child. A characteristic finding of placental abruption is known as plate hardness (persistent hardness of the uterus). Accordingly, for example, an evaluation index regarding the possibility of placental abruption can be obtained using the intensity H and duration L of the uterine contractions, or a function or the like including the intensity H and duration L as elements.

The evaluation information 4B based on the fetal heartbeat includes, for example, an evaluation of the health status of the fetus FE and the signs or possibility of intrauterine fetal death. As an example, the health status of the fetus FE can be evaluated by an indicator of the presence or absence or number of transient tachycardia (a temporary increase in the fetal heart rate B) within a certain period of time as an indicator of evaluation. From the inferred pregnancy status 3, when the transient tachycardia is observed a predetermined number of times within a determination period, the status of the fetus FE is evaluated to be good (reactive). When no transient tachycardia is observed, the status of the fetus FE is evaluated to be abnormal (non-reactive). The abnormal cases vary; some cases are not particularly problematic, while others are signs of intrauterine fetal death. The overall evaluation information 4C can include information such as an evaluation regarding the need for a visit to a medical institution from the information 3A on the uterine contractions and the information 3B on the fetal heartbeat. This is expected to contribute to early medical intervention for important signs and improved treatment plans for perinatal complications, as well as reduce the burden on pregnant women and corresponding medical personnel by reducing unnecessary visits when the pregnancy status 3 is good.

### Generation of Learned Model

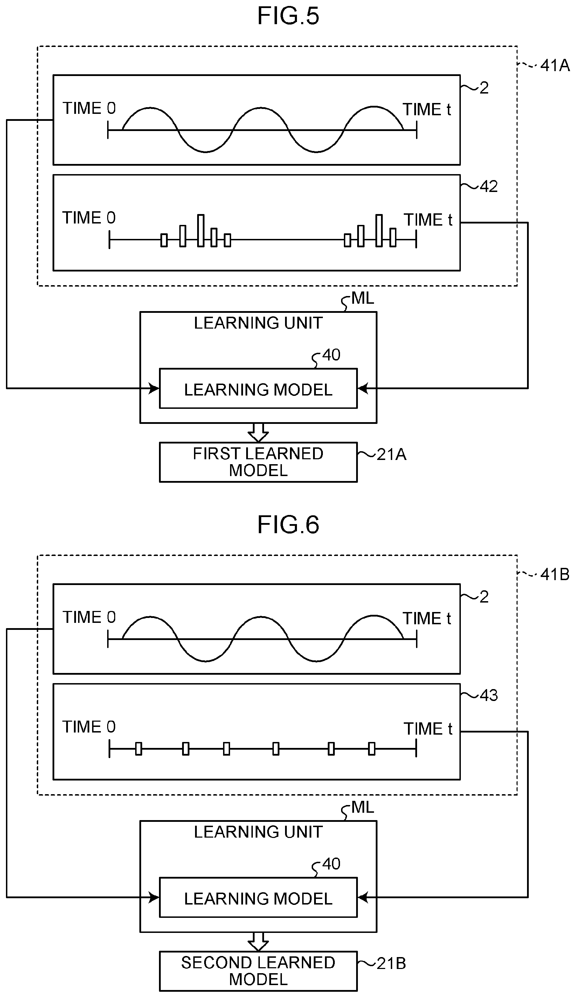

FIG. 5 is an explanatory diagram for explaining the generation (machine learning) of the first learned model 21A. FIG. 6 is an explanatory diagram for explaining the generation (machine learning) of the second learned model 21B. The learning algorithms for the learned model 21 (the first learned model 21A and the second learned model 21B) are not particularly limited, but include, for example, any one of a convolutional neural network (CNN), a recurrent neural network (RNN), a long short-term memory (LSTM), and an attention mechanism (including a transformer with an attention mechanism), or combinations thereof.

As illustrated in FIGS. 5 and 6, the learned model 21 is generated by a learning unit ML, which is a computer that performs machine learning, performing the machine learning of the learning model 40 by using teacher data. The teacher data includes data actually measured from pregnant women who cooperate in the generation of the teacher data. The teacher data can be generated by measurement data using dedicated devices such as childbirth monitoring devices, for example, at hospitals.

As illustrated in FIG. 5, teacher data 41A used to generate the first learned model 21A includes the mother body-derived electrical activity record 2 (time-series data) measured percutaneously and a uterine contraction record 42 measured from the same mother body. The uterine contraction record 42 is time-series data on the intensity of uterine contractions. The electrical activity record 2 and the uterine contraction record 42 included in the teacher data 41A are pairs of data measured at the same time t. The uterine contraction record 42 is information corresponding to the information 3A on the uterine contractions generated in the inference unit 11. The learning unit ML uses the teacher data 41A to cause the learning model 40 to acquire, by using deep learning, features to be converted into the output of the uterine contraction record 42 when the mother body-derived electrical activity record 2 measured percutaneously is input. The features are weights or parameters for each node of each layer (an output layer, an input layer, and a hidden layer) constituting the learning model 40. The learning unit ML determines the features of the learning model 40 by performing learning using a plurality of teacher data 41A. The learning model 40 whose features have been determined by machine learning is the first learned model 21A.

The teacher data 41A may be data for a specific period of time (number of weeks) during the pregnancy, or data for all weeks from conception. By using, as the teacher data 41A, uterine contraction data obtained from pregnant women at all gestational weeks, which covers threatened miscarriage (from conception to less than 22 weeks), threatened premature birth (from 22 weeks to less than 37 weeks), and placental abruption (22 weeks or later), the information 3A on the uterine contractions of the mother body MO that is output can be expected to contribute in various ways, such as early intervention from threatened miscarriage to threatened premature birth, uterine rigidity in placental abruption, and prediction of the onset of labor pain. By using data for a specific period of time (number of weeks) as the teacher data 41A, machine learning specialized for a specific purpose such as detecting signs of threatened premature birth, for example, can be performed.

The teacher data 41A may be measurement data (what is called raw data) using a dedicated device such as a childbirth monitoring device, or data annotated on the measurement data. The annotation is a process of adding information to data (measurement data) used for learning. Examples of the annotation include specifying time information at the start and end of uterine contractions in the measurement data. This allows for efficient learning of conditions in which effective uterine contractions are occurring and conditions in which no effective uterine contractions are occurring.

As illustrated in FIG. 6, teacher data 41B used to generate the second learned model 21B includes the mother body-derived electrical activity record 2 (time-series data) measured percutaneously and a fetal heartbeat record 43 measured from the same mother body. The fetal heartbeat record 43 is time-series data on the intensity of fetal heartbeat. The electrical activity record 2 and the fetal heartbeat record 43 included in the teacher data 41B are pairs of data measured at the same time t. The fetal heartbeat record 43 is information corresponding to the information 3B on the fetal heartbeat generated in the inference unit 11. The learning unit ML uses the teacher data 41B to cause the learning model 40 to acquire, by using deep learning, features to be converted into the output of the fetal heartbeat record 43 when the mother body-derived electrical activity record 2 measured percutaneously is input. The learning unit ML determines the features of the learning model 40 by performing learning using a plurality of teacher data 41B. The learning model 40 whose features have been determined by machine learning is the second learned model 21B.

The teacher data 41B may be data for a specific period of time (number of weeks) or data for all periods of time from the minimum number of weeks for which a fetal heartbeat can be detected by Doppler/echo. The minimum number of weeks for which a fetal heartbeat can be detected is usually about five weeks. According to learning using, as the teacher data 41B, data from the minimum number of weeks for which a heartbeat can be detected, the second learned model 21B that can accommodate changes in the heartbeat component due to the fetal growth process is obtained.

As described above, when the preprocessing is applied to the electrical activity record 2 that is input to the learned model 21 in the inference unit 11, the same preprocessing is also applied to teacher data used for learning. Accordingly, the electrical activity record 2 used as the teacher data may be filtered to remove predetermined signal components corresponding to the respiratory fluctuations of the mother body MO. The electrical activity record 2 used as the teacher data may be electrocardiogram data that has been preprocessed to extract information that identifies at least one of the baseline, P-point, Q-point, R-point, S-point, and T-point.

The following is a description of the inference of the uterine contractions and the fetal heartbeat of the mother body MO from the mother body MO-derived electrical activity record 2 measured percutaneously. Human biological activities such as muscle activity and brain activity are caused by electrical stimulation. Therefore, electrical signals measured percutaneously from the mother body MO (electrical activity record 2) include various signal components associated with biological activities, including signal components derived from the uterine contractions and signal components derived from the fetal heartbeat. The learning process illustrated in FIGS. 5 and 6 is to learn a process of separating and extracting the signal components derived from the uterine contractions and the heartbeat components derived from the fetus, from the time-series data of the electrical activity record 2, which includes such various signal components. The signal components derived from the uterine contractions include not only electrical signals derived from uterine smooth muscle, but also electrical signals generated by other biological activities associated with the occurrence of the uterine contractions (for example, electrical signals generated from muscles other than uterine muscle due to stiffening of the mother body, axis deviation of the electrocardiogram due to diaphragm changes associated with uterine contractions, changes in heart rate due to pain stimulation associated with uterine contractions, and the like).

For example, the following Non Patent Literature discloses a technique for detecting arrhythmia patients from electrocardiogram signals of patients in sinus rhythm by deep learning, as a technique for detecting diseases and the like of a subject by machine learning from electrical signals acquired percutaneously from the subject.

Zachi I Attia, Peter A Noseworthy, Francisco Lopez-Jimenez, Samuel J Asirvatham, Abhishek J Deshmukh, Bernard J Gersh, et al. "An artificial intelligence-enabled ECG algorithm for the identification of patients with atrial fibrillation during sinus rhythm: a retrospective analysis of outcome prediction", Lancet. 2019 (published online Aug 1), Vol 394, Issu 10201, p. 861-867, [online] URL<https://doi.org/10.1016/50140-6736(19)31721-0>

The learned model 21 (the first learned model 21A and the second learned model 21B) generated in this way is stored in advance in the storage unit 14 of the pregnancy status estimation device 1 illustrated in FIG. 1.

### Pregnancy Status Estimation Method

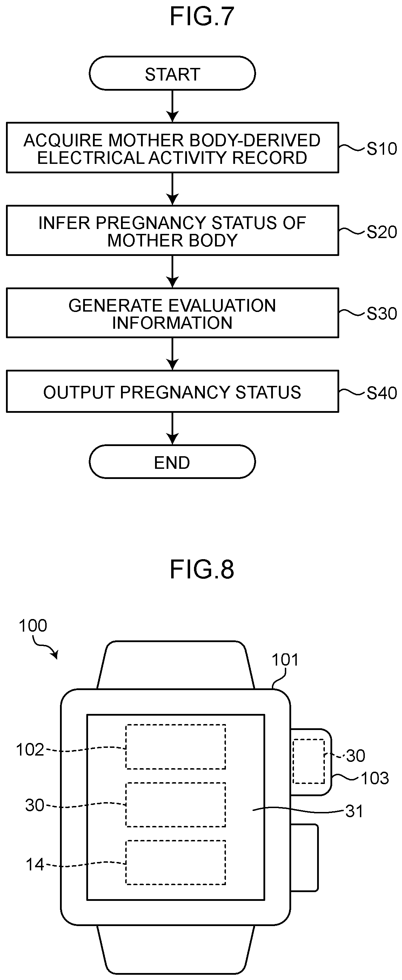

The pregnancy status estimation method according to the first embodiment is described below. FIG. 7 is a flowchart for explaining the pregnancy status estimation method according to the first embodiment. The pregnancy status estimation method is also a method of operating the pregnancy status estimation device 1.

The pregnancy status estimation method includes step S10 of acquiring the mother body MO-derived electrical activity record 2 measured percutaneously, step S20 of inferring the pregnancy status 3 of the mother body MO by inputting the electrical activity record 2 into the learned model 21 that receives the electrical activity record 2 as input and outputs the pregnancy status 3 including information on at least one of the uterine contractions of the mother body MO and the fetal heartbeat in the mother body MO, and step S40 of outputting the pregnancy status 3. The pregnancy status estimation method according to the first embodiment further includes step S30 of generating the evaluation information 4 according to the content of the pregnancy status 3 generated by the inference unit 11.

At step S10, the information acquisition unit 10 acquires the mother body MO-derived electrical activity record 2 measured percutaneously. The information acquisition unit 10 acquires the electrical activity record 2 from the sensor 30 attached to the mother body MO. The information acquisition unit 10 may acquire the electrical activity record 2 by reading data from a storage medium (for example, a flash memory, an external server, or the like) that stores the electrical activity record 2 after the electrical activity record 2 is measured by the sensor 30 attached to the mother body MO.

At step S20, the inference unit 11 infers the pregnancy status 3 of the mother body MO by inputting the electrical activity record 2 into the learned model 21. The inference unit 11 inputs the electrical activity record 2 acquired at step S10 into the first learned model 21A and the second learned model 21B, to perform calculations based on respective features thereof. In this way, the inference unit 11 generates the pregnancy status 3 including the information 3A on the uterine contractions of the mother body MO and the information 3B on the fetal heartbeat in the mother body MO.

At step S30, the evaluation unit 12 generates the evaluation information 4 on the basis of the data on the pregnancy status 3 and the evaluation data 22. In the first embodiment, the evaluation unit 12 generates the evaluation information 4A based on the uterine contractions, the evaluation information 4B based on the fetal heartbeat, and the overall evaluation information 4C.

At step S40, the output unit 13 outputs, to an output destination device, output data including the pregnancy status 3 generated by the inference unit 11 and the evaluation information 4 generated by the evaluation unit 12. The output destination device is the display unit 31 in the first embodiment. This causes the pregnancy status 3 and the evaluation information 4 to be displayed on the display unit 31.

### Configuration Example of Pregnancy Status Estimation Device

The pregnancy status estimation device 1 described above can be implemented in various forms other than the example illustrated in FIG. 1.

FIG. 8 is an explanatory diagram illustrating a first configuration example of the pregnancy status estimation device 1 according to the first embodiment. In the example of FIG. 8, the pregnancy status estimation device 1 is implemented by a wearable device 100. The wearable device 100 is a small device that can be worn on a part of a body. The wearable device 100 has a band (strip) shape that can be worn, for example, on the wrist, ankle, upper arm, thigh, or the like. FIG. 8 illustrates an example where the wearable device 100 is a smart watch (wristwatch-type information terminal worn on the wrist).

The wearable device 100 includes a control unit 102, a storage unit 14, a display unit 31, and a sensor 30. The control unit 102, the storage unit 14, the display unit 31, and the sensor 30 are incorporated in one enclosure 101.

The control unit 102 is composed of a processor such as a CPU. The processor functions as the control unit 102 that controls the wearable device 100 by performing arithmetic processing in accordance with a computer program stored in the storage unit 14. The storage unit 14 stores the computer program 20 of the pregnancy status estimation device 1. The information acquisition unit 10, the inference unit 11, the evaluation unit 12, and the output unit 13 of the pregnancy status estimation device 1 are configured by the control unit 102 provided in the wearable device 100 and executing the computer program 20.

The storage unit 14 includes a nonvolatile semiconductor memory. The storage unit 14 stores computer programs (operation system or firmware) for controlling the wearable device 100 and the computer program 20 (application program) of the pregnancy status estimation device 1. The storage unit 14 also stores the learned model 21 and the evaluation data 22 illustrated in FIG. 1.

The display unit 31 includes a self-illuminating display device such as a liquid crystal display device or an organic electroluminescence (EL) display device. The display unit 31 displays various information on the wearable device 100 under the control of the control unit 102.

The sensor 30 percutaneously measures electrical signals (changes in an electric field) generated by the biological activity of a wearer by means of electrodes that contact the wearer. The sensor 30 is, for example, an electrical heart rate sensor. In the example of FIG. 8, the sensor 30 includes a first electrode provided on the inner peripheral surface of the enclosure 101 that is in contact with the wearer's wrist, and a second electrode provided on an operating unit 103 provided on the lateral surface of the enclosure 101. When a pregnant woman wears the wearable device 100 on the wrist of one hand (for example, left hand), the mother body MO is in contact with the first electrode. During measurement, when the pregnant woman touches the operating unit 103 with her other hand (right hand), the mother body MO is in contact with the second electrode. In this example, the sensor 30 can measure a signal corresponding to the I induction of an electrocardiogram, which measures the potential difference between the left hand and the right hand.

With this configuration, the information acquisition unit 10 acquires the electrical activity record 2 from the sensor 30 that is provided in the wearable device 100 and percutaneously measures an electrical activity. The generation of the pregnancy status 3 by the inference unit 11 and the generation of the evaluation information 4 by the evaluation unit 12 are implemented by arithmetic processing performed by the control unit 102 provided in the wearable device 100 and executing the computer program 20. The output unit 13 outputs the pregnancy status 3 and the evaluation information 4 to the display unit 31 provided in the wearable device 100. The display unit 31 displays the pregnancy status 3 and the evaluation information 4 supplied from the output unit 13. In this way, the pregnancy status estimation device 1 can be implemented by the wearable device 100. In other words, the wearable device 100 performs the pregnancy status estimation method according to the first embodiment by executing the computer program 20.

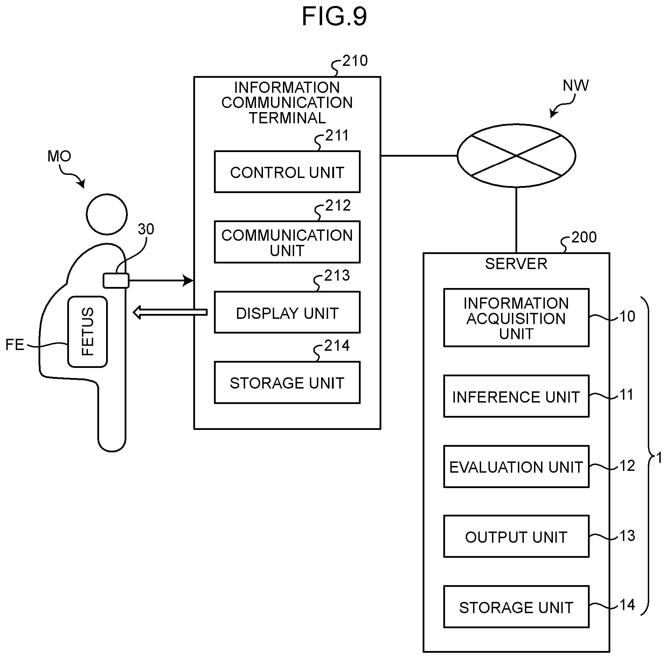

FIG. 9 is an explanatory diagram illustrating a second configuration example of the pregnancy status estimation device 1 according to the first embodiment. In the example of FIG. 9, the pregnancy status estimation device 1 is implemented by a server 200 connected to a network NW. The pregnancy status 3 and the evaluation information 4 generated by the pregnancy status estimation device 1 is provided to a user in the form of what is called a cloud service.

The server 200 is a computer that can communicate via the network NW. The server 200 includes an information acquisition unit 10, an inference unit 11, an evaluation unit 12, an output unit 13, and a storage unit 14. The information acquisition unit 10, the inference unit 11, the evaluation unit 12, and the output unit 13 of the pregnancy status estimation device 1 are implemented on the computer program 20 when the server 200 executes the computer program 20 (see FIG. 1) stored in the storage unit 14. The server 200 may be configured by a single device or a group of a plurality of devices (server group). For example, the server 200 may be configured by a server device functioning as the information acquisition unit 10, a server device functioning as the inference unit 11, a server device functioning as the evaluation unit 12, a server device functioning as the storage unit 14, and the like, and the pregnancy status estimation device 1 may be configured by intercommunication between these server devices. The output unit 13 is implemented by the communication function of the server device.

In the example of FIG. 9, a user, who is a pregnant woman or her family, uses the pregnancy status estimation device 1 by using an information communication terminal 210 that can communicate with the server 200 via the network NW. The information communication terminal 210 can be a smart phone, a tablet-type terminal, a personal computer (PC), or the like. The information communication terminal 210 includes a control unit 211, a communication unit 212, a display unit 213, and a storage unit 214. The storage unit 214 stores an application program for using the functions of the pregnancy status estimation device 1 through communication with the server 200. The control unit 211 acquires, via the communication unit 212, the mother body MO-derived electrical activity record 2 measured percutaneously from the sensor 30 attached to the mother body MO. The control unit 211 establishes communication with the server 200 via the communication unit 212 by executing the application program, and transmits the electrical activity record 2 to the server 200.

At the server 200, the information acquisition unit 10 acquires the electrical activity record 2 transmitted from the information communication terminal 210. The inference unit 11 inputs the electrical activity record 2 into the learned model 21 stored in the storage unit 14 and infers the pregnancy status 3. The evaluation unit 12 generates the evaluation information 4 on the basis of the pregnancy status 3. The output unit 13 outputs the pregnancy status 3 and the evaluation information 4 to the information communication terminal 210 via the network NW.

The control unit 211 of the information communication terminal 210 receives the pregnancy status 3 and the evaluation information 4 from the server 200 via the communication unit 212. The control unit 211 displays the received pregnancy status 3 and evaluation information 4 on the display unit 213. In this way, the pregnancy status estimation device 1 is implemented by the server 200 that provides cloud services. In other words, the server 200 performs the pregnancy status estimation method according to the first embodiment by executing the computer program 20.

### Effects

As described above, the pregnancy status estimation device 1 according to the first embodiment includes: the information acquisition unit 10 that acquires the mother body MO-derived electrical activity record 2 measured percutaneously; the inference unit 11 that infers the pregnancy status 3 of the mother body MO by inputting the electrical activity record 2 to the learned model 21 that receives the electrical activity record 2 as input and outputs the pregnancy status including information on at least one of the uterine contractions of the mother body MO and the fetal heartbeat in the mother body MO; and the output unit 13 that outputs the pregnancy status 3 generated by the inference unit 11. This allows the pregnancy status 3 to be inferred and provided to a user simply by percutaneously measuring the mother body MO-derived electrical activity record 2, without the use of an expensive and large dedicated device such as a childbirth monitoring device. The user can ascertain the objective pregnancy status 3 generated by the inference process without requiring expertise or data interpretation work to decipher the electrical activity record 2. As a result, the status of a mother body or a fetus can be ascertained inexpensively and easily even outside a hospital.

In the pregnancy status estimation device 1 according to the first embodiment, the learned model 21 includes the first learned model 21A that outputs the information on the uterine contractions of the mother body MO and the second learned model 21B that outputs the information on the fetal heartbeat in the mother body MO, and the inference unit 11 infers the pregnancy status 3 including each of the information 3A on the uterine contractions of the mother body MO and the information 3B on the fetal heartbeat in the mother body MO. This allows both the information 3A on the uterine contractions of the mother body MO and the information 3B on the fetal heartbeat in the mother body MO to be provided to the user. As a result, motivation can be provided for user behavior that leads to early medical intervention to suppress premature birth and intrauterine fetal death. In addition, by separately providing the first learned model 21A and the second learned model 21B, a learning process can be performed more easily than when a single learned model is used to infer both the information 3A on the uterine contractions and the information 3B on the fetal heartbeat.

The pregnancy status estimation device 1 according to the first embodiment further includes the evaluation unit 12 that generates the evaluation information 4 according to the content of the pregnancy status 3 generated by the inference unit 11, and the output unit 13 outputs the evaluation information 4 according to the pregnancy status 3 in addition to the pregnancy status 3. This allows the user to be provided with information on evaluations derived from the pregnancy status 3 in addition to objective information on the pregnancy status 3. This allows the user without expertise to more easily ascertain the status of the mother body MO and the fetus FE.

In the pregnancy status estimation device 1 according to the first embodiment, the information 3A on the uterine contractions of the mother body MO includes at least one of the following information: intensity, duration, interval, cycle, frequency, and intensity waveform of the uterine contractions. This allows objective data on the uterine contractions of the mother body MO to be obtained.

In the pregnancy status estimation device 1 according to the first embodiment, the information 3B on the fetal heartbeat in the mother body MO includes at least one of the following information: fetal heartbeat, fetal heart rate, fetal heart sound, heartbeat interval, heart sound waveform, and findings of the fetal heartbeat. This allows objective data on the fetal heartbeat in the mother body MO to be obtained.

In the pregnancy status estimation device 1 according to the first embodiment, the information acquisition unit 10, the inference unit 11, and the output unit 13 are configured by the control unit 102 provided in the wearable device 100 that can be worn on a part of a body and executing the computer program 20. The information acquisition unit 10 acquires the electrical activity record 2 from the sensor 30 provided in the wearable device 100 and percutaneously measuring an electrical activity, and the output unit 13 outputs the pregnancy status 3 to the display unit 31 provided in the wearable device 100. This allows the wearable device 100 having sensing and display functions to implement everything from measuring the electrical activity record 2 to displaying the pregnancy status 3. In the case of using the wearable device 100 available on the market, the pregnancy status estimation device 1 can be provided at a noticeably lower cost than dedicated devices such as childbirth monitoring devices, and can be easily installed even outside a hospital.

The pregnancy status estimation method according to the first embodiment comprises: step S10 of acquiring the mother body MO-derived electrical activity record 2 measured percutaneously, step S20 of inferring the pregnancy status 3 of the mother body MO by inputting the electrical activity record 2 to the learned model 21 that receives the electrical activity record 2 as input and outputs the pregnancy status 3 including information on at least one of the uterine contractions of the mother body MO and the fetal heartbeat in the mother body MO, and step S40 of outputting the pregnancy status 3. This allows the pregnancy status 3 to be inferred from the mother body MO-derived electrical activity record 2 measured percutaneously and to be provided to the user, without the use of an expensive and large dedicated device such as a childbirth monitoring device. The user can ascertain the objective pregnancy status 3 generated by the inferential process without requiring expertise in deciphering the electrical activity record 2. As a result, the status of a mother body or a fetus can be ascertained inexpensively and easily even outside a hospital.

### Second Embodiment

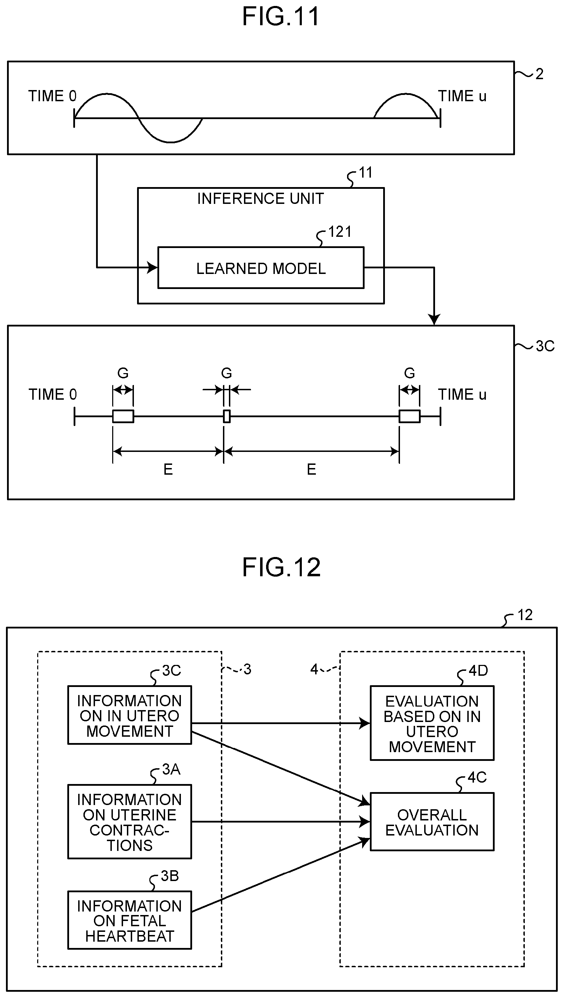

A second embodiment is described below. The second embodiment describes an example in which the inference unit 11 infers a pregnancy status 3 including information 3C (see FIG. 11) on an in utero movement of the fetus FE in the mother body MO. In the second embodiment, parts that share the same configuration with the first embodiment are omitted from the explanation.

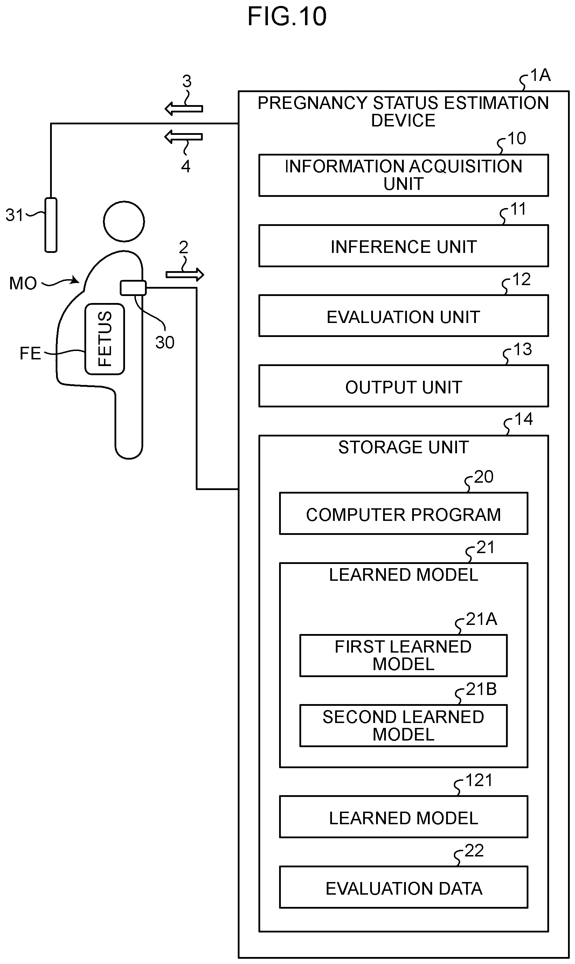

FIG. 10 is a schematic block diagram illustrating a pregnancy status estimation device according to the second embodiment. As illustrated in FIG. 10, a pregnancy status estimation device 1A according to the second embodiment includes an information acquisition unit 10, an inference unit 11, an evaluation unit 12, an output unit 13, and a storage unit 14.

In the second embodiment, the storage unit 14 stores a learned model 121 that outputs the pregnancy status 3 including the information 3C (see FIG. 11) on the in utero movement of the fetus FE in the mother body MO. In the example of FIG. 10, the storage unit 14 also stores the learned model 21 according to the first embodiment above.

In the second embodiment, the information acquisition unit 10 acquires the electrical activity record 2 via a sensor 30 that contacts one or more of the limbs of the mother body MO and detects electrical signals. The sensor 30 is a sensor that detects electrical signals used for electrocardiogram or electromyogram. The sensor 30 is placed on the surface of one of the limbs of the mother body MO, that is, right arm, left arm, right leg, and left leg. The sensor 30 percutaneously measures electrical signals generated by a biological activity of the mother body MO by means of electrodes that contact the mother body MO. The placement location of the sensor 30 is not particularly limited, but may be any part from the shoulder to the fingertips of the hand in the case of the right or left arm. The placement location of the sensor 30 may be any part from the base of the leg to the toes in the case of the right or left leg. The sensor 30 may be located at a plurality of parts of the limbs of the mother body MO. In the second embodiment, the sensor 30 is a sensor built into a wearable sensor device or a multifunctional wearable device (see FIG. 8). In the case of the wearable device 100 illustrated in FIG. 8, by wearing the wearable device 100 on the wrist of one hand (for example, left hand), the mother body MO and the first electrode of the sensor 30 are in contact with each other. During measurement, when the pregnant woman touches the operating unit 103 with her other hand (right hand), the mother body MO is in contact with the second electrode. The pregnant woman may allow the operating unit 103 to contact a body surface other than the limbs, such as the surface of the chest or abdominal wall. The sensor 30 measures the potential difference between contact parts of the first electrode and the second electrode. The information acquisition unit 10 acquires data that is measured while the pregnant woman wears the sensor 30 in her daily life. The sensor 30 may be placed on a body surface other than the limbs of the mother body MO. For example, in the case of the wearable device 100 illustrated in FIG. 8, the first electrode provided on the inner peripheral surface of the enclosure 101 is placed on the abdominal wall surface of the mother body MO, and the pregnant woman touches the operating unit 103 with either hand to bring the mother body MO and the second electrode in contact with each other. This also makes it possible to acquire the electrical activity record 2.

In the second embodiment, the inference unit 11 infers the pregnancy status 3 by using the learned model 121. That is, the inference unit 11 infers the pregnancy status 3 of the mother body MO by inputting the electrical activity record 2 into the learned model 121 that receives the electrical activity record 2 as input and outputs the pregnancy status 3 including the information 3C on the in utero movement of the fetus FE in the mother body MO. The output pregnancy status 3 includes numerical information on the in utero movement of the fetus FE in the mother body MO. In the example of FIG. 10, the inference unit 11 uses the first learned model 21A to infer the information 3A on the uterine contractions of the mother body MO, and uses the second learned model 21B to infer the information 3B on the fetal heartbeat in the mother body MO. In the second embodiment, the pregnancy status 3 includes the information 3A on the uterine contractions, the information 3B on the fetal heartbeat, and the information 3C on the in utero movement of the fetus FE. The pregnancy status 3 of the second embodiment may not include the information 3A on the uterine contractions and the information 3B on the fetal heartbeat. As in the first embodiment, the inference unit 11 may preprocess the electrical activity record 2. The inference unit 11 may perform preprocessing on the electrical activity record 2, including a filtering process of removing predetermined signal components corresponding to the respiratory fluctuations of the mother body MO. The inference unit 11 may perform preprocessing to add information identifying at least one of the baseline, P-point, Q-point, R-point, S-point, and T-point to the electrical activity record 2 being electrocardiogram data.

FIG. 11 is an explanatory diagram illustrating the learned model 121 according to the second embodiment. The learned model 121 is pre-generated by machine learning so that the mother body MO-derived electrical activity record 2 is input and the information 3C on the in utero movement of the fetus FE in the mother body MO is output. The electrical activity record 2 as teacher data may be preprocessed data.

In the present specification, the movement of the fetus refers to the movement (motion) of the fetal body in utero and is a broad concept that encompasses the muscle tone, fetal movement, and respiratory-like movement of the fetus. The movement of the fetus specifically refers to the temporary movement of the fetus and includes no physiological activities of internal organs that are constantly taking place, such as the fetal heartbeat.

The information 3C on the in utero movement of the fetus FE in the mother body MO includes at least one of the following information: muscle tone, fetal movement, and respiratory-like movement of the fetus FE. The muscle tone of the fetus FE is a flexion and extension movement of a part (trunk or limbs) of a body of the fetal FE. Examples of the muscle tone include the movement of the spine or limbs from a flexed position to an extended position and back to the original flexed position, and the opening and closing of the palms. The fetal movement of the fetus FE is a single or combined movement of the trunk or limbs of the fetus FE, but refers to greater movement than the muscle tone. Examples of the fetal movement of the fetus FE include changes in body orientation and kicking at the uterine wall. The respiration-like movement of the fetus FE is similar to breathing. Examples of the respiration-like movement include intermittent movements of the diaphragm, abdominal wall, and thorax of the fetal FE that last for several seconds to several tens of seconds.

In FIG. 11, the learned model 121 outputs the information 3C on the in utero movement of the fetus FE in the mother body MO as time-series data indicating the time of occurrence of the in utero movement. In FIG. 11, the information 3C on the in utero movement is indicated by a one-dimensional graph in which a horizontal axis represents time (time u) and the occurrence of the in utero movement is represented by the presence or absence of bar plots. The abscissa length of the bar plot represents the duration from the start to the end of one in utero movement. Locations where no bar plots are present indicate that no in utero movement has occurred. In the example of FIG. 11, the information 3C on the in utero movement includes an interval E of the in utero movement. The interval E of the in utero movement is a time interval between one in utero movement and the next in utero movement. The information 3C on the in utero movement may include a duration G of the in utero movement. The duration G of the in utero movement is a duration from the start to the end of one in utero movement. The information 3C on the in utero movement may be time-series data including information on the intensity of the in utero movement. In such a case, the information 3C on the in utero movement can be represented by a two-dimensional graph in which a vertical axis represents the intensity of the in utero movement and a horizontal axis represents time (time u). The learned model 121 may generate the information 3C on the in utero movement that distinguishes the muscle tone, fetal movement, and respiratory-like movement from one another, or may generate the information 3C on the in utero movement without distinguishing the muscle tone, fetal movement, and respiratory-like movement from one another.

FIG. 12 is an explanatory diagram for explaining evaluation information according to the second embodiment. The evaluation unit 12 generates evaluation information 4 on the basis of the data on the pregnancy status 3 and evaluation data 22 (see FIG. 10) stored in advance in the storage unit 14. In the second embodiment, the evaluation unit 12 generates evaluation information 4D based on the in utero movement of the fetus FE on the basis of the information 3C on the in utero movement.

The evaluation information 4D based on the in utero movement includes, for example, an evaluation of the frequency of the in utero movement (the length of the interval E of the in utero movement). A correlation exists between the frequency of the in utero movement of the fetus FE and the health of the FE, and a decrease in frequency or loss of the in utero movement (what is called decreased fetal movement) is an indicator of signs or possibility of intrauterine fetal death or placental abruption. The evaluation information 4D based on the in utero movement includes, for example, an evaluation of whether the interval E of the in utero movement reaches a predetermined criterion (that is, whether the fetal movement is decreasing). The predetermined criterion is, for example, a relative criterion that the interval E is equal to or greater than N% (N>100) compared to the average of the most recent M hours. The frequency of the in utero movement per unit time may be calculated from the interval E, and \compared with the criterion. The evaluation information 4D based on the in utero movement may include, for example, an evaluation of whether the duration G of the in utero movement exceeds the predetermined criterion (that is, an evaluation of the length of one in utero movement).

In the second embodiment, the information 3C on the in utero movement is routinely and continuously acquired by the wearable sensor 30, and the evaluation information 4D based on the in utero movement is generated by the evaluation unit 12. The information 3C on the in utero movement and the evaluation information 4D based on the in utero movement enable physicians and others to ascertain objective data and evaluations over a relatively long period of time to determine whether the fetal movement is decreasing.

In the example of FIG. 12, the evaluation unit 12 generates overall evaluation information 4C on the basis of not only the information 3C on the in utero movement of the fetus FE, but also the information 3A on the uterine contractions and the information 3B on the fetal heartbeat described in the first embodiment. The evaluation information 4 generated by the evaluation unit 12 is supplied to the output unit 13. The output unit 13 outputs, to the display unit 31, output data including the pregnancy status 3 generated by the inference unit 11 and the evaluation information 4 generated by the evaluation unit 12.

### Generation of Learned Model

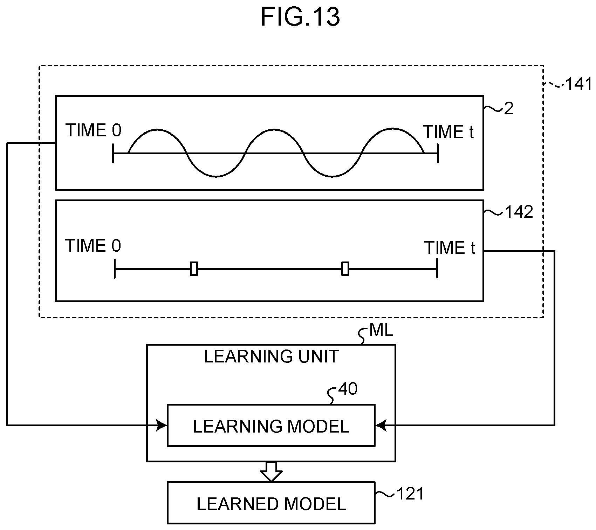

FIG. 13 is an explanatory diagram for explaining the generation (machine learning) of the learned model 121. In the second embodiment, teacher data 141 used for machine learning of the learning model 40 is generated by measurement data using a dedicated device such as a childbirth monitoring device and an ultrasound inspection device (echo or Doppler), for example, at hospitals.

The teacher data 141 used to generate the learned model 121 includes the mother body-derived electrical activity record 2 (time-series data) measured percutaneously and an in utero movement record 142 of the fetus FE measured from the fetus FE in the same mother body. The in utero movement record 142 is the time-series data of the in utero movement of the fetus FE in the mother body MO. The electrical activity record 2 and the in utero movement record 142 included in the teacher data 141 are pairs of data measured at the same time t. The in utero movement record 142 is acquired by placing the probe of the ultrasound inspection device or the ultrasound transducer of the childbirth monitoring device on the surface of the abdominal wall of the mother body MO, measuring acoustic signals, and extracting signal components derived from the fetus FE through arithmetic processing. The in utero movement record 142 is information corresponding to the information 3C on the in utero movement of the fetus FE generated in the inference unit 11. The learning unit ML uses the teacher data 141 to cause the learning model 40 to acquire, by using deep learning, features to be converted into the output of the in utero movement record 142 when the mother body-derived electrical activity record 2 measured percutaneously is input. The learning unit ML determines the features of the learning model 40 by performing learning using a plurality of teacher data 141. The learning model 40 whose features have been determined by machine learning is the learned model 121.

The in utero movement of the fetus FE is accompanied by a much larger bioelectrical signal than the fetal heartbeat described in the first embodiment above. Bioelectrical signals derived from the in utero movement, which is estimated as the information 3C on the in utero movement, can be indirectly measured from the fetus FE through the mother body MO. The bioelectrical signals derived from the in utero movement include not only electrical signals from the fetal movement itself, but also biological activities of the mother body associated with the occurrence of the fetal movement (such as electrical signals derived from the rectus abdominis and biceps brachii muscles due to stiffening of the mother body), and these biological activities of the mother body are independent of conscious or unconscious activities. The learned model 121 acquires, by machine learning, a process of separating and extracting signal components derived from the in utero movement of the fetus FE from the electrical activity record 2 of the mother body MO. The generated learned model 121 is stored in advance in the storage unit 14 of the pregnancy status estimation device 1 illustrated in FIG. 10.

### Pregnancy Status Estimation Method

The pregnancy status estimation method according to the second embodiment is described below. The flow of processing for the pregnancy status estimation method according to the second embodiment is similar to the flowchart illustrated in FIG. 7, with the only difference being the content of processing for some of the steps.

At step S10, the information acquisition unit 10 acquires the mother body MO-derived electrical activity record 2 measured percutaneously. The information acquisition unit 10 acquires the electrical activity record 2 directly from the sensor 30 attached to the mother body MO or via a storage medium that records the measurement data of the sensor 30. The information acquisition unit 10 acquires the electrical activity record 2 by the wearable sensor 30. In one measurement, for example, for a pregnant woman who is the subject of the measurement and is in a semi-seated position and at rest, the electrical activity record 2 is measured for about 20 to 40 minutes. The measurement is performed, for example, when the pregnant woman feels a "tight stomach" or "little fetal movement", but may also be performed periodically, for example, at a specific time each day.

At step S20, the inference unit 11 infers the pregnancy status 3 of the mother body MO by inputting the electrical activity record 2 into the learned model 21. In the second embodiment, the inference unit 11 performs feature-based calculations by inputting the electrical activity record 2 acquired at step S10 into the learned model 121 that receives the electrical activity record 2 as input and outputs the pregnancy status 3 including the information 3C on the in utero movement of the fetus FE in the mother body MO. In this way, the inference unit 11 infers the pregnancy status 3 including the information 3C on the in utero movement of the fetus FE. In the second embodiment, the inference unit 11 further inputs the electrical activity record 2 acquired at step S10 into the first learned model 21A and the second learned model 21B to perform calculations based on respective features. In this way, the inference unit 11 generates the pregnancy status 3 including the information 3A on the uterine contractions of the mother body MO and the information 3B on the fetal heartbeat in the mother body MO, in addition to the information 3C on the in utero movement.

At step S30, the evaluation unit 12 generates the evaluation information 4 on the basis of the data on the pregnancy status 3 and the evaluation data 22. In the second embodiment, the evaluation unit 12 generates the evaluation information 4D based on the in utero movement, the evaluation information 4A based on the uterine contractions, the evaluation information 4B based on the fetal heartbeat, and the overall evaluation information 4C.

At step S40, the output unit 13 outputs output data including the pregnancy status 3 generated by the inference unit 11 and the evaluation information 4 generated by the evaluation unit 12 to an output destination device such as the display unit 31. This causes the pregnancy status 3 and the evaluation information 4 to be displayed on the display unit 31.

### Configuration Example of Pregnancy Status Estimation Device

The pregnancy status estimation device 1A according to the second embodiment can be implemented in various forms other than the example illustrated in FIG. 10, as in the first embodiment above. The pregnancy status estimation device 1A according to the second embodiment is implemented, for example, by the wearable device 100 illustrated in FIG. 8. The pregnancy status estimation device 1A according to the second embodiment can be implemented, for example, by the server 200 illustrated in FIG. 9, and can provide the pregnancy status 3 and the evaluation information 4 in the form of a cloud service.

### Effects

As described above, the pregnancy status estimation device 1A according to the second embodiment includes: the information acquisition unit 10 that acquires the mother body MO-derived electrical activity record 2 measured percutaneously; the inference unit 11 that infers the pregnancy status 3 of the mother body MO by inputting the electrical activity record 2 to the learned model 121 that receives the electrical activity record 2 as input and outputs the pregnancy status 3 including information 3C on the in utero movement of the fetus FE in the mother body MO; and the output unit 13 that outputs the pregnancy status 3 generated by the inference unit 11. This allows the pregnancy status 3 to be inferred and provided to a user simply by percutaneously measuring the mother body MO-derived electrical activity record 2, without the use of an expensive and large dedicated device such as a childbirth monitoring device or an ultrasound inspection device. The user can ascertain the objective pregnancy status 3 generated by the inference process without requiring expertise or data interpretation work to decipher the electrical activity record 2. As a result, the status of the fetus FE can be ascertained inexpensively and easily even outside a hospital.

The in utero movement of the fetus FE can be measured even outside a hospital, for example, by fixing a dedicated sensor on the surface of the abdominal wall of a pregnant woman and using an ultrasound inspection device. However, since such a sensor and a data processing device are expensive and difficult to install and the sensor fixed to the abdominal wall surface makes it difficult to continue daily life or perform long-term measurement, sufficiently acquiring measurement data outside a hospital is difficult. Therefore, in the related art, the in utero movement of the fetus FE was mostly ascertained through interviews with pregnant women who visited hospitals for routine outpatient visits and the information ascertained was the pregnant woman's subjective impression of a sense of decreased fetal movement, making it difficult to use as a useful indicator for ascertaining signs of intrauterine fetal death or placental abruption. According to the second embodiment, physicians and others can ascertain objective data to determine the presence or absence of a decrease in the fetal movement by the information 3C on the in utero movement, so that contribution to early medical intervention and improved treatment plans for intrauterine fetal death or placental abruption can be expected.

In the pregnancy status estimation device 1A according to the second embodiment, the information 3C on the in utero movement of the fetus FE in the mother body MO includes at least one of the following information: muscle tone, fetal movement, and respiratory-like movement of the fetus FE. This makes it possible to ascertain objective data that serves as an indicator of the health of the fetal FE.

In the pregnancy status estimation device 1A according to the second embodiment, the information acquisition unit 10 acquires the electrical activity record 2 via the sensor 30 that contacts one or more of the limbs of the mother body MO to detect electrical signals. This makes it possible to acquire the electrical activity record 2 simply by attaching, for example, the wearable sensor 30 to an arm or a leg, unlike the method in the related art of fixing a dedicated sensor to the surface of the abdominal wall to ascertain the status of the fetus FE. For example, in the case of the sensor 30 provided in the wearable device 100 illustrated in FIG. 8, as described above, since the first electrode of the sensor 30 worn on the wrist of one hand makes contact with the mother body MO, the pregnant woman touches the operating unit 103 with the other hand or allows the operating unit 103 to contact the surface of the chest or abdominal wall during measurement, so that measurement can be performed. Since the pregnant woman only needs to touch the operating unit 103, she can remain clothed, and even when the operating unit 103 contacts the chest or abdominal wall, she can easily take measurements simply by placing her hand inside her clothing. Therefore, there are fewer restrictions on the posture, movements, clothing, and the like of the pregnant woman wearing the sensor 30 during measurement, so that continuous and high-frequency measurement in her daily life can be performed without compromising quality of life (QOL) of the pregnant woman. As a result, the possibility of detecting important signs related to intrauterine fetal death or placental abruption can be increased.

Other effects of the second embodiment are the same as the first embodiment above.

Although the embodiments of the present invention has been described, the embodiments are not limited by the content of these embodiments. The aforementioned components also include those that can be readily assumed by those skilled in the art, those that are substantially identical, and those that are in what is called range of equivalence. Furthermore, the aforementioned components can be combined as appropriate. Furthermore, various omissions, substitutions, or modifications of the components can be made without departing from the gist of the aforementioned embodiments.

### Fine Tuning

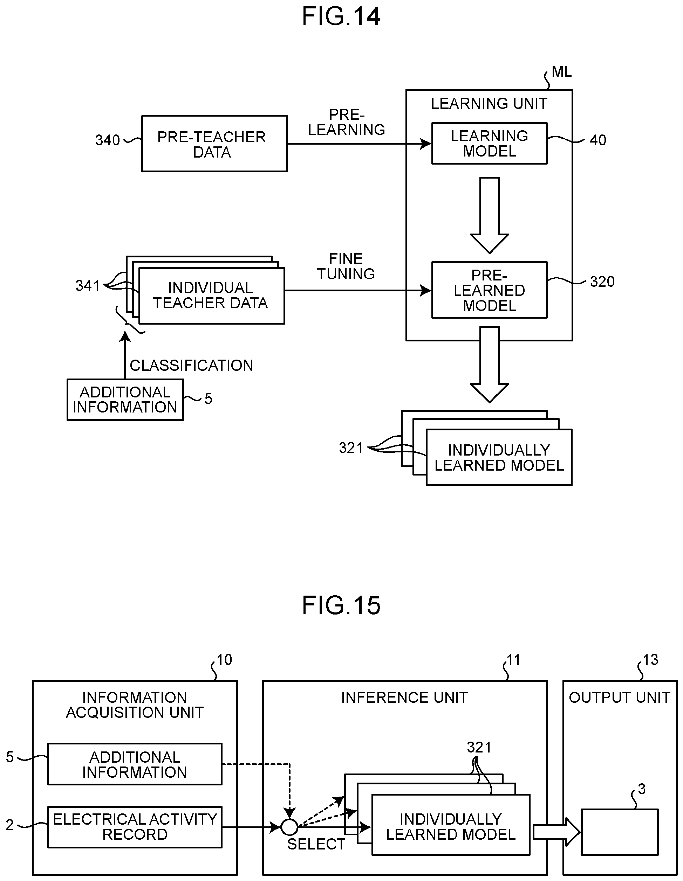

For example, the learned model 21 (121) used in the first and second embodiments above may be fine-tuned based on additional information on the pregnancy status 3 of the mother body MO or the fetus FE. FIG. 14 is a schematic diagram for explaining the generation of individually learned models by fine tuning according to a modification. FIG. 15 is a schematic diagram for explaining an inference process using the individually learned models according to the modification.

As illustrated in FIG. 14, the learned model 21 includes a plurality of individually learned models 321 obtained by fine-tuning a pre-learned model 320 by using individual teacher data 341 of the electrical activity record 2 and the pregnancy status 3 classified on the basis of additional information 5. The individually learned model 321 is generated by fine-tuning the pre-learned model 320, which has been learned in advance using the learning model 40 by pre-teacher data 340, by the individual teacher data 341. Hereafter, the learned model fine-tuned by the individual teacher data 341 is referred to as the individually learned model 321.

The additional information 5 is information on the pregnancy status 3 of the mother body MO or the fetus FE, other than the electrical activity record 2. The additional information 5 includes at least one of the following: gestational weeks, estimated fetal weight, maternal body mass index (BMI), maternal height, maternal weight, and maternal age. By means of the additional information 5, the teacher data of the electrical activity record 2 and the pregnancy status 3 are classified into one of a plurality of groups. The individual teacher data 341, which is a group of teacher data belonging to one of the groups of the additional information **5,** is generated for each group.

The learning model 40 is machine-learned (pre-learned) by the pre-teacher data 340 that is not grouped (randomly including data from all groups), so that the pre-learned model 320 is generated. Some or all of the generated pre-learned model 320 are learned by fine tuning with the individual teacher data 341. As a result, the individually learned models 321 are generated for each group of the additional information **5.** The number of data points in the individual teacher data 341 may be fewer than the number of data points in the pre-teacher data 340.

The method of classifying (grouping) the individual teacher data 341 is not limited. The following is an example where the additional information 5 is the gestational weeks. The additional information 5 on the gestational weeks may be classified into three groups according to the gestational weeks, for example, early pregnancy (before 13 weeks and 6 days), mid-pregnancy (14 weeks and 0 days to 27 weeks and 6 days), and late pregnancy (28 weeks and 0 days and later). The individual teacher data 341 is composed of teacher data obtained from the mother body MO whose gestational weeks belongs to one of the respective groups. Subsequently, through fine tuning using each of individual teacher data 341 for early pregnancy, individual teacher data 341 for mid-pregnancy, and individual teacher data 341 for late pregnancy, an individually learned model 321 for early pregnancy, an individually learned model 321 for mid-pregnancy, an individually learned model 321 for late pregnancy are generated separately.

As illustrated in FIG. 15, the information acquisition unit 10 acquires the additional information 5 on the pregnancy status 3 of the mother body MO or the fetus FE, in addition to the electrical activity record 2. The additional information 5 (or information necessary for calculating the additional information 5) may be input to the pregnancy status estimation device 1 by a user such as the mother body MO or a physician (for example, transmitted from an information communication terminal), or may be acquired by the information acquisition unit 10 from electronic medical record information or the like. The gestational weeks as an example can be calculated, for example, from the date of conception of the mother body MO and the current date, and the date of conception of the mother body MO is acquired by the method above.

The inference unit 11 selects an individually learned model 321 into which the electrical activity record 2 is input, on the basis of the additional information 5 acquired by the information acquisition unit 10. By inputting the electrical activity record 2 into the selected individually learned model 321, the inference unit 11 generates the pregnancy status 3 of the mother body MO and supplies the generated pregnancy status 3 to the output unit 13.

This allows the individually learned model 321 for inference to be selected according to which of the three groups the gestational weeks of the mother body MO fall into: early pregnancy, mid-pregnancy, and late pregnancy. As a result, the accuracy of inferences according to the gestational weeks can be improved. The frequency of uterine contractions and the frequency and intensity of fetal movement of a fetus tend to increase with advancing gestational weeks, and fine tuning according to gestational weeks is effective in improving inference accuracy. In addition, the electrical activity record 2 such as electrocardiograms varies from the statistical mean depending on a subject's body size (maternal BMI, maternal height, and maternal weight). Fine tuning according to the maternal BMI, maternal height, maternal weight, and the like can address statistical biases in input data, which is effective in improving inference accuracy. The frequency and intensity of fetal movements tend to increase as gestational weeks progress and the estimated fetal weight increases. Therefore, fine tuning according to estimated fetal weight as well as gestational week is effective in improving inference accuracy. In addition, increasing maternal age increases the possibility that the intensity of uterine contractions will decrease, and against this background, older births are at risk for weak labor and delivery arrest. Fine tuning according to age allows tuning that takes into account the degree of weakening of uterine contraction, contributing to improved inference accuracy.

No particular limitation exists in the number of groups into which the additional information 5 is classified. The gestational weeks may also be classified into two or four or more groups, or into separate groups for each gestational week. The same is true for other additional information **5.** In addition to the aforementioned gestational weeks, estimated fetal weight, maternal BMI, maternal height, maternal weight, and maternal age, the additional information 5 may also include the presence or absence of previous pregnancy or childbirth, race, nationality, life history (smoking, alcohol consumption, and occupational history), pregnancy history (natural pregnancy, artificial insemination, and in vitro fertilization), medical history, obstetric complications, internal medicine, allergic history, family history, presence or absence of edema, urine test findings (urine protein and urine sugar), and the like.

Although FIG. 14 illustrates an example in which fine tuning based on the additional information 5 is performed on the pre-learned model 320, the individually learned model 321 may be generated (without fine tuning) by separately performing, on the learning model 40, machine learning based on the additional information 5. That is, learned models 21 for each classification based on the additional information 5 may be separately generated by performing machine learning on the learning model 40 by using the individual teacher data 341 classified on the basis of the additional information 5.

### Reference Signs List

- 1, 1A: Pregnancy status estimation device
- 2: Electrical activity record
- 3: Pregnancy status
- 3A: Information on uterine contractions
- 3B: Information on fetal heartbeat
- 3C: Information on in utero movement
- 4: Evaluation information
- 4A: Evaluation information based on uterine contractions
- 4B: Evaluation information based on fetal heartbeat
- 4C: Overall evaluation information
- 4D: Evaluation information based on in utero movement
- 5: Additional information
- 10: Information acquisition unit
- 11: Inference unit
- 12: Evaluation unit
- 13: Output unit
- 14: Storage unit
- 20: Computer program
- 21: Learned model
- 21A: First learned model
- 21B: Second learned model
- 22: Evaluation data
- 30: Sensor
- 31: Display unit
- 40: Learning model
- 41A, 41B: Teacher data
- 42: Uterine contraction record
- 43: Fetal heartbeat record
- 100: Wearable device
- 101: Enclosure
- 102: Control unit
- 103: Operating unit
- 121: Learned model
- 141: Teacher data
- 142: In utero movement record
- 200: Server
- 210: Information communication terminal
- 211: Control unit
- 212: Communication unit
- 213: Display unit
- 214: Storage unit
- 320: Pre-learned model
- 321: Individually learned model
- 340: Pre-teacher data
- 341: Individual teacher data
- B: Fetal heart rate
- D: Interval of uterine contractions
- E: Interval of in utero movement
- FE: Fetus
- G: Duration of in utero movement
- H: Intensity of uterine contractions
- L: Duration of uterine contractions
- ML: Learning unit
- MO: Mother body
- NW: Network

## Claims

1. A pregnancy status estimation device comprising:
an information acquisition unit that acquires a mother body-derived electrical activity record measured percutaneously;
an inference unit that infers a pregnancy status of a mother body by inputting the electrical activity record into a learned model that receives the electrical activity record as input and outputs the pregnancy status including information on at least one of uterine contractions of the mother body and a fetal heartbeat in the mother body; and
an output unit that outputs the pregnancy status generated by the inference unit.

2. The pregnancy status estimation device according to claim 1, wherein
the learned model includes
a first learned model that outputs information on the uterine contractions of the mother body, and
a second learned model that outputs information on the fetal heartbeat in the mother body, and
the inference unit infers the pregnancy status including each of the information on the uterine contractions of the mother body and the information on the fetal heartbeat in the mother body.

3. The pregnancy status estimation device according to claim 1 or 2, further comprising:
an evaluation unit that generates evaluation information according to content of the pregnancy status generated by the inference unit, wherein
the output unit outputs the evaluation information according to the pregnancy status, in addition to the pregnancy status.

4. The pregnancy status estimation device according to claim 1 or 2, wherein the information on the uterine contractions of the mother body includes information on at least one of intensity, duration, interval, cycle, frequency, and intensity waveform of the uterine contractions.

5. The pregnancy status estimation device according to claim 1 or 2, wherein the information on the fetal heartbeat in the mother body includes information on at least one of fetal heartbeat, fetal heart rate, fetal heart sound, heartbeat interval, heart sound waveform, and findings of the fetal heartbeat.

6. The pregnancy status estimation device according to claim 1 or 2, wherein
the information acquisition unit, the inference unit, and the output unit are configured by a control unit provided in a wearable device wearable on a part of a body and executing a computer program,
the information acquisition unit acquires the electrical activity record from a sensor provided in the wearable device and percutaneously measuring an electrical activity, and
the output unit outputs the pregnancy status to a display unit provided in the wearable device.

7. A pregnancy status estimation method comprising:
a step of acquiring a mother body-derived electrical activity record measured percutaneously,
a step of inferring a pregnancy status of the mother body by inputting the electrical activity record to a learned model that receives the electrical activity record as input and outputs the pregnancy status including information on at least one of uterine contractions of a mother body and a fetal heartbeat in the mother body, and
a step of outputting the pregnancy status.

8. A pregnancy status estimation device comprising:
an information acquisition unit that acquires a mother body-derived electrical activity record measured percutaneously;
an inference unit that infers a pregnancy status of a mother body by inputting the electrical activity record into a learned model that receives the electrical activity record as input and outputs the pregnancy status including information on an in utero movement of a fetus in the mother body; and
an output unit that outputs the pregnancy status generated by the inference unit.

9. The pregnancy status estimation device according to claim 8, wherein the information on the in utero movement of the fetus in the mother body includes information on at least one of muscle tone, fetal movement, and respiratory-like movement of the fetus.

10. The pregnancy status estimation device according to claim 8, wherein the information acquisition unit acquires the electrical activity record via a sensor that contacts at least one of limbs of the mother body and detects electrical signals.

11. The pregnancy status estimation device according to any one of claims 8 to 10, further comprising:
an evaluation unit that generates evaluation information according to content of the pregnancy status generated by the inference unit, wherein
the output unit outputs the evaluation information according to the pregnancy status, in addition to the pregnancy status.

12. A pregnancy status estimation method comprising:
a step of acquiring a mother body-derived electrical activity record measured percutaneously;
a step of inferring a pregnancy status of a mother body by inputting the electrical activity record into a learned model that receives the electrical activity record as input and outputs the pregnancy status including information on an in utero movement of a fetus in the mother body; and
a step of outputting the pregnancy status.
